(19) Europäisches Patentamt
European Patent Office
Office européen des brevets

(11) Veröffentlichungsnummer: **0 423 072 A2**

# (12) EUROPÄISCHE PATENTANMELDUNG

(21) Anmeldenummer: **90810764.2**

(22) Anmeldetag: **04.10.90**

(51) Int. Cl.[5]: **C07D 333/36**, A01N 43/10

(30) Priorität: **13.10.89 CH 3750/89**

(43) Veröffentlichungstag der Anmeldung: **17.04.91 Patentblatt 91/16**

(84) Benannte Vertragsstaaten: **BE CH DE DK ES FR GB GR IT LI LU NL**

(71) Anmelder: **CIBA-GEIGY AG**
**Klybeckstrasse 141**
**CH-4002 Basel(CH)**

(72) Erfinder: **Pascual, Alfons, Dr.**
**Gundeldingerstrasse 433**
**CH-4053 Basel(CH)**

## (54) Thienylthioharnstoffe, -isothioharnstoffe und -carbodiimide.

(57) Die neuen substituierten 3-Thienylthioharnstoffe, -isothioharnstoffe und -carbodiimide der Formel I

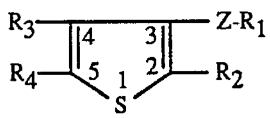

(I),

worin

$R_1$ $C_1$-$C_{12}$-Alkyl, durch einen $C_3$-$C_6$-Cycloalkylrest substituiertes $C_1$-$C_4$-Alkyl, $C_3$-$C_8$-Cycloalkyl oder durch einen $C_1$-$C_4$-Alkylrest substituiertes $C_3$-$C_6$-Cycloalkyl;

$R_2$, $R_3$ und $R_4$ unabhängig voneinander Wasserstoff, $C_1$-$C_6$-Alkyl, Phenyl oder durch 1 bis 3 Reste aus der Gruppe Halogen, $C_1$-$C_6$-Alkyl, $C_1$-$C_4$-Alkoxy und $C_1$-$C_6$-Halogenalkyl mit 1 bis 9 Halogenatomen substituiertes Phenyl;

Z eine Gruppe -NH-CS-NH-, -N = C($SR_5$)-NH- oder -N = C = N-; und

$R_5$ $C_1$-$C_6$-Alkyl oder $C_3$-$C_5$-Alkenyl;

bedeuten; mit der Massgabe, dass mindestens einer der Reste $R_2$, $R_3$ oder $R_4$ eine von Wasserstoff abweichende Bedeutung hat; sowie die Salze von Verbindungen der Formel I, worin Z die Gruppe -N = C($SR_5$)-NH- bedeutet, besitzen wertvolle pestizide Eigenschaften. Diese Verbindungen enthaltende Mittel, Verfahren zu ihrer Herstellung sowie ihre Verwendung als Pestizide, insbesondere als Insektizide und Akarizide in der Landwirtschaft, werden beschrieben.

EP 0 423 072 A2

## THIENYLTHIOHARNSTOFFE, -ISOTHIOHARNSTOFFE UND -CARBODIIMIDE

Die vorliegende Erfindung betrifft neue substituierte 3-Thienylthioharnstoffe, -isothioharnstoffe und -carbodiimide, Salze dieser Verbindungen mit organischen und anorganischen Säuren, Verfahren und Zwischenprodukte zu ihrer Herstellung, Schädlingsbekämpfungsmittel, die diese Verbindungen enthalten, sowie ihre Verwendung bei der Kontrolle von Schädlingen.

Erfindungsgemäss werden Verbindungen der Formel I vorgeschlagen

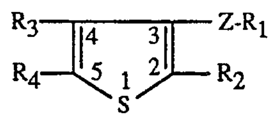

(I),

worin

$R_1$ $C_1$-$C_{12}$-Alkyl, durch einen $C_3$-$C_6$-Cycloalkylrest substituiertes $C_1$-$C_4$-Alkyl, $C_3$-$C_8$-Cycloalkyl oder durch einen $C_1$-$C_4$-Alkylrest substituiertes $C_3$-$C_6$-Cycloalkyl;

$R_2$, $R_3$ und $R_4$ unabhängig voneinander Wasserstoff, $C_1$-$C_6$-Alkyl, Phenyl oder durch 1 bis 3 Reste aus der Gruppe Halogen, $C_1$-$C_6$-Alkyl, $C_1$-$C_4$-Alkoxy und $C_1$-$C_6$-Halogenalkyl mit 1 bis 9 Halogenatomen substituiertes Phenyl;

Z eine Gruppe -NH-CS-NH-, -N = C($SR_5$)-NH- oder -N = C = N-; und

$R_5$ $C_1$-$C_6$-Alkyl oder $C_3$-$C_5$-Alkenyl;

bedeuten; mit der Massgabe, dass mindestens einer der Reste $R_2$, $R_3$ oder $R_4$ eine von Wasserstoff abweichende Bedeutung hat; sowie die Salze von Verbindungen der Formel I, worin Z die Gruppe -N = C-($SR_5$)-NH- bedeutet.

Bei den als Substituenten in Betracht kommenden Halogenen handelt es sich sowohl um Fluor und Chlor als auch um Brom und Jod, wobei Fluor und Chlor bevorzugt sind.

Die als Substituenten auch von anderen Gruppen in Betracht kommenden Alkylreste können geradkettig oder verzweigt sein. Als Beispiele solcher Alkyle seien Methyl, Aethyl, Propyl, i-Propyl, Butyl, i-Butyl, sek.Butyl, tert.Butyl oder Pentyl, Hexyl, Octyl usw. und ihre Isomeren genannt.

Die als Substituenten in Betracht kommenden Alkenylreste können geradkettig oder verzweigt sein und eine oder mehrere Doppelbindungen aufweisen. Beispiele solcher Alkenyle sind u.a. Allyl, 1-Propenyl, Isopropenyl, Allenyl, Butenyle oder Pentenyle.

Die als Substituenten im Rahmen der erfindungsgemässen Definition in Betracht kommenden ein- oder mehrfach durch Halogen substituierten $C_1$-$C_6$-Alkylreste können geradkettig oder verzweigt und nur teilweise oder auch perhalogeniert sein, wobei für die Halogene die oben gegebenen Definitionen gelten. Geeignete Beispiele solcher Substituenten sind u.a. das ein- bis dreifach durch Fluor, Chlor und/oder Brom substituierte Methyl, wie z.B. $CHF_2$ oder $CF_3$; das ein- bis fünffach durch Fluor, Chlor und/oder Brom substituierte Aethyl, wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder i-Propyl, wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren, wie z.B. $CF(CF_3)CHFCF_3$ oder $CH_2(CF_2)_2CF_3$.

Bei den als Substituenten in Betracht kommenden Cycloalkylresten handelt es sich beispielsweise um Cyclopropyl, Cyclobutyl, Cyclopentyl, Cyclohexyl oder Cyclooctyl. Die Cycloalkyle können im Rahmen der erfindungsgemässen Definition ein- oder mehrfach durch einen $C_1$-$C_4$-Alkylrest substituiert und/oder über eine $C_1$-$C_4$-Alkylenbrücke mit dem Rest des Moleküls verbunden sein.

Die Verbindungen der Formel I, in denen Z für -N = C($SR_5$)-NH- steht, d.h. die Isothioharnstoffe der Formel I, können auch in Form ihrer Säureadditionssalze vorliegen. Zur Bildung solcher Salze eignen sich sowohl organische als auch anorganische Säuren. Beispiele solcher Säuren sind u.a. Chlorwasserstoffsäure, Bromwasserstoffsäure, Jodwasserstoffsäure, Salpetersäure, verschiedene Phosphorsäuren, Schwefelsäure, Ameisensäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure, vorzugsweise Oxalsäure.

Verbindungen der Formel I, in denen Z für -N = C($SR_5$)-NH- steht, können in den tautomeren Formen entsprechend den Strukturen -N = C($SR_5$)-NH- ⇄ -NH-C($SR_5$) = N- vorliegen. Die Erfindung umfasst sowohl die einzelnen Tautomeren, als auch Tautomerengemische.

Unter den Verbindungen der Formel I stehen aufgrund ihrer biologischen Wirkungen diejenigen im Vordergrund, worin mindestens zwei der Reste $R_2$, $R_3$ oder $R_4$ eine von Wasserstoff abweichende Bedeutung haben.

Ferner sind solche Verbindungen der Formel I bevorzugt, in denen $R_4$ Wasserstoff bedeutet, sowie solche, in denen $R_1$ $C_1$-$C_4$-Alkyl oder $C_3$-$C_5$-Cycloalkyl, speziell $C_4H_9$(t) oder $C_3H_7$(i) bedeutet.

Von besonderer Bedeutung sind diejenigen Verbindungen der Formel I, worin $R_2$ und $R_3$ unabhängig voneinander $C_2$-$C_5$-Alkyl bedeuten, diejenigen, worin $R_2$ und/oder $R_3$ $C_3H_7$(i) bedeuten, sowie diejenigen, worin $R_5$ Methyl oder Äthyl bedeutet.

Die erfindungsgemässen Verbindungen der Formel I können nach im Prinzip bekannten Verfahren (vgl. z.B. EP Patentanmeldung 0.304.025) hergestellt werden, indem man z.B.

a) zur Herstellung einer Verbindung der Formel I, worin Z für -NH-CS-NH- steht, eine Verbindung der Formel II

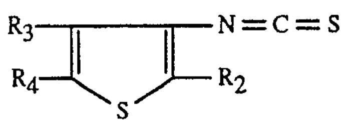

(II)

mit einer Verbindung der Formel III

$H_2N$-$R_1$ (III)

umsetzt; oder

b) gegebenenfalls zur Herstellung einer Verbindung der Formel I, worin Z für -N=C($SR_5$)-NH- steht, die erhaltene Verbindung der Formel I, worin Z für -NH-CS-NH-steht, mit einer Verbindung der Formel IV

X-$R_1$ (IV)

umsetzt; und dass man gewünschtenfalls eine erhaltene Verbindung der Formel I, worin Z für -N=C-($SR_5$)-NH- steht, in an sich bekannter Weise in eines ihrer Salze überführt; oder

c) gegebenenfalls zur Herstellung einer Verbindung der Formel I, worin Z für -N=C=N-steht, aus einer erhaltenen Verbindung der Formel I, worin Z für -NH-CS-NH- steht, Schwefelwasserstoff abspaltet;

wobei in den Formeln II bis IV $R_1$ bis $R_5$ die oben angegebenen Bedeutungen haben und X für eine Abgangsgruppe steht.

Verfahren a) wird üblicherweise unter normalem Druck, und in Gegenwart eines organischen Lösungs- oder Verdünnungsmittels durchgeführt. Die Temperatur beträgt dabei zwischen 0 und +150°C, vorzugsweise +10 bis 70°C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Verfahren b) wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid. Die Reaktion wird entweder in Gegenwart einer Base durchgeführt oder aber das allfällig gebildete Salz anschliessend einer basischen Nachbehandlung unterworfen. (vgl. J.B. Hendricksen et al., "Organic Chemistry", Mc Graw Hill Book Co., 1970, p. 378-382).

Geeignete Abgangsgruppen in Ausgangsverbindungen der Formel IV sind z.B. Halogenatome, insbesondere Chlor, Brom oder Jod, oder gegebenenfalls halogenierte oder alkylierte Sulfonsäureester, wie z.B. Tosylat, Brosylat oder Mono- oder Dialkylsulfat (Mesylat, Dimethylsulfat).

Verfahren c) wird zweckmässigerweise in einem aprotischen organischen Lösungs- oder Verdünnungsmittel unter normalem Druck durchgeführt. Die Temperatur beträgt dabei zwischen 0 und +150°C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6 , 1873, p. 1398; G. Amiard, Bull. Soc. chim. 1956, p. 1360). Als Abspaltungs-Reagenzien werden dabei z.B. HgO, bestimmte Pyridinium Salze, Chloressigsäureester, Cyanursäurechlorid, p-Toluolsulfochlorid oder bestimmte Phosphorsäureester-Derivate verwendet.

Die als Ausgangsprodukte verwendeten Isothiocyanate der Formel II sind neu. Sie können nach im Prinzip bekannten Methoden hergestellt werden, z.B. indem man ein Anilin der Formel V

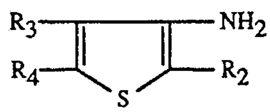

(V),

mit Thiophosgen umsetzt, wobei $R_2$, $R_3$ und $R_4$, die oben unter Formel I angegebenen Bedeutungen haben (vgl. Helv. Chem. Acta 66 , 148; EP-Patentanmeldung 043.054).

Das Verfahren zur Herstellung der obigen Verbindungen der Formel II wird zweckmässigerweise in Gegenwart einer organischen oder anorganischen Base und einem gegenüber den Reaktionsteilnehmern inerten Lösungs- oder Verdünnungsmittels, bei einer Temperatur zwischen 0 und + 100 °C und bei normalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder chlorierte Kohlenwasserstoffe, wie Dichlormethan oder Tetrachlormethan. Geeignete Basen können organischen oder anorganischen Ursprungs sein, wie z.B. Natriumhydrid, Natrium- oder Calciumcarbonat, tertiäre Amine wie Triäthylamin, Triäthylendiamin oder 4-Dimethylaminopyridin oder Pyridin.

Die vorerwähnten Aniline der Formel V sind bekannt bzw. können nach im Prinzip bekannten Methoden hergestellt werden, indem man z.B. ein entsprechendes Oxim (vgl. Synthesis 1977 , 200) unter Umwandlung der Oxim- in die Aminogruppe aromatisiert (vgl. JP Patentpublikation 69-12.895; J. Org. Chem. 1953 , 138; Arch. Pharm. 314 , 557).

Die Ausgangsverbindungen der Formeln III und IV sind zumeist bekannt und können nach im Prinzip bekannten Methoden hergestellt werden.

Es ist bereits aus den deutschen Offenlegungsschriften Nr. 2657772, 2727416 und 2727529 bekannt, dass substituierte N-Phenyl-N′-alkyl- bzw. -N′-cycloalkyl-thioharnstoffe akarizide und/oder insektizide Wirkung besitzen. Ferner umfasst die EP-Patentanmeldung Nr. 0300.972 insektizid und akarizid wirksame substituierte N-Thienyl-N′-benzoylharnstoffe und -thioharnstoffe, wobei der N-[4-Methyl-5-(4-chlorphenyl)-thien-3-yl]-N′-(2,6-difluorbenzoyl)-thioharnstoff dort spezifisch genannt wird. Demgegenüber unterscheiden sich die erfindungsgemässen Verbindungen der Formel I strukturell im wesentlichen durch das gleichzeitige Vorliegen einer N-Thien-3-yl sowie einer N′-Alkyl-Gruppierung und andererseits auch durch das Fehlen einer N′-Benzoylgruppierung.

Es wurde nun überraschenderweise gefunden, dass die erfindungsgemässen Verbindungen der Formel I bei günstiger Warmblüter-, Fisch- und Pflanzenverträglichkeit wertvolle Wirkstoffe in der Schädlingsbekämpfung darstellen. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor, insbesondere an Haus- und Nutztieren, vorkommen. Die Verbindungen der Formel I sind speziell geeignet zur Bekämpfung von pflanzenschädigenden saugenden Insekten und Spinnmilben sowie von im Boden auftretenden Schädlingen, wie z.B. Diabrotica balteata. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp.,

Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.; aus der Ordnung der Orthoptera zum Beispiel Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.; aus der Ordnung der Mallophaga zum Beispiel Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die erwähnte gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise

verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)- Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykolläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"1985 International Mc Cutcheon's Emulsifiers & Detergents", Glen Rock NJ USA, 1985",
"H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die erfindungsgemässen pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte erfindungsgemässe Formulierungen folgendermassen zusammen:
(% = Gewichtsprozent)

Emulgierbare Konzentrate:
Aktiver Wirkstoff: 1 bis 20 %, bevorzugt 5 bis 10 %
oberflächenaktives Mittel: 5 bis 30 %, vorzugsweise 10 bis 20 %
flüssiges Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspension-Konzentrate:
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %

Benetzbare Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermaterial: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Die erfindungsgemässen Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel 1: Herstellung von 2,4-Diisopropyl-3-thienylisothiocyanat (Ausgangsverbindung)

5,60 g 2,4-Diisopropylthienyl-3-amin-hydrochlorid werden unter Rühren in kleinen Portionen bei 0° bis 5°C zu einem Gemisch aus 5,50 g Calciumcarbonat, 40 ml Wasser, 3,75 g Thiophosgen, 70 ml Dichlormethan und 2,70 g Natriumbicarbonat gegeben. Das Reaktionsgemisch wird 3 Stunden lang bei Raumtemperatur gerührt und dann über Kieselgur filtriert. Die organische Phase wird abgetrennt, mit 50 ml gesättigter NaCl-Lösung und 50 ml Wasser gewaschen, über Magnesiumsulfat getrocknet und anschliessend eingedampft. Die Titelverbindung der Formel

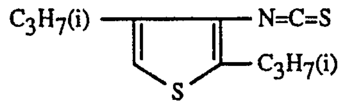

liegt als stark gefärbtes Öl vor, das ohne weitere Reinigung weiterverwendet wird.

In analoger Weise werden aus den angegebenen Thienyl-3-aminen die nachfolgend aufgeführten Thienyl-3-isocyanate der Formel II hergestellt:

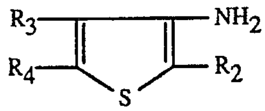

| $R_2$ | $R_3$ | $R_4$ | phys. Daten/$^1$H-NMR (60 MHz, $CDCl_3$) |
|---|---|---|---|
| $CH_3$ | $CH_3$ | $CH_3$ | 3,30 br s(2); 2,20 s(3); 2,15 s(3); 1,95 s(3) |
| $CH_3$ | H | $C_6H_5$ | 7,6-7,1 m(5); 6,70 s(1); 3,25 br s(2); 2,25 s(3) |
| $CH_3$ | $C_3H_7$-i | $C_4H_9$-i | 3,55 br s(2); 3,05 hept(1); 2,50 d(2) J=7, 2,10 s(3); 1,9-1,6 m(1); 1,25 d(6) J=7; 0,90 d(6) J=7 |
| i-$C_3H_7$ | H | $C_6H_5$ | 7,5-7,1 m(5); 6,65 s(1); 3,30 br s(2); 3,00 hept (1) J=7; 1,20 d(6) J=7 |
| i-$C_3H_7$ | H | $CH_3$ | 6,15 q(1) J=1; 3,15 br s(2); 2,95 hept (1) J=7; 2,30 d(3) J=1; 1,20 d(6) J=7 |
| i-$C_3H_7$ | $CH_3$ | $CH_3$ | 3,90 br s(2); 3,00 hept (1) J=7; 2,20 s(3), 1,90 s(3); 1,20 d(6) J=7 |
| i-$C_3H_7$ | $C_3H_7$-i | H | 6,55 s(1), 3,55 br s(2); 3,05 hept (1) J=7, 2,75 hept (1) J=7; 1,25 d(6) J=7; 1,20 d(6) J=7 |
| $C_2H_5$ | $C_3H_7$-i | H | 6,65 s(1), 2,73 hept(1) J=7, 2,52 q(2) J=7, 1,55 bs(2), 1,28 t(3) J=7, 1,25 d(6) J=7 |

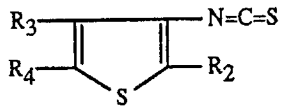

(II)

| $R_2$ | $R_3$ | $R_4$ | phys. Daten/$^1$H-NMR (60 MHz, $CDCl_3$) |
|---|---|---|---|
| H | H | $C_6H_5$ | 7,50-7,15 m(5); 7,05 d(1) J=1,5; 6,95 d(1) J=1,5 |
| H | H | $CH_3$ | 6,85 d(1) J=1; 6,60 m(1); 2,40 d(3) J=1 |
| H | $CH_3$ | $CH_3$ | 6,85 s(1); 2,30 s(3); 2,10 s(3) |
| $CH_3$ | $CH_3$ | $CH_3$ | 2,30 s(6); 2,10 s(3) |
| $CH_3$ | H | $C_6H_5$ | 7,50-7,20 m(5); 6,95 s(1); 2,45 s(3) |
| $CH_3$ | $C_3H_7$-i | $C_4H_9$-i | 3,05 hept (1) J=7; 2,50 d(2) J=7; 2,30 s(3), 1,90-1,60 m(1); 1,25 d(6) J=7; 0,90 d(6) J=7 |
| $C_3H_7$-i | H | $C_6H_5$ | 7,50-7,15 m(5); 6,85 s(1); 3,30 hept (1) J=7; 1,30 d(6) J=7 |
| $C_3H_7$-i | H | $CH_3$ | 6,45 d(1) J=1; 3,25 hept (1) J=7; 2,35 br s(3); 1,30 d(6) J=7 |
| $C_3H_7$-i | $CH_3$ | $CH_3$ | 3,25 hept (1) J=7; 2,25 s(3); 2,05 s(3), 1,20 d(6) J=7 |
| $C_3H_7$-i | $C_3H_7$-i | H | 6,60 s(1), 3,35 hept (1) J=7; 2,95 hept (1) J=7; 1,30 d(6) J=7; 1,25 d(6) J=7 |
| $C_2H_5$ | $C_3H_7$-i | H | 6,68 s(1), 2,94 hept (1) J=7, 2,85 q(2) J=7, 1,29 t(3) J=7, 1,24 d(6) J=7 |

Beispiel 2: Herstellung von 1-tert.Butyl-3-(2,4-diisopropyl-3-thienyl)thioharnstoff

5,75 g des gemäss Beispiel 1 hergestellten 2,4-Diisopropylthienyl-3-isothiocyanats werden mit 70 ml Toluol verdünnt und tropfenweise unter Rühren mit 2,16 g tert.Butylamin versetzt. Anschliessend wird bei ca. +80 °C während 2 Stunden weitergerührt. Das Reaktionsgemisch wird eingeengt und der Rückstand

mit Hexan versetzt. Der entstandene Festkörper wird abfiltriert, mit Hexan nachgewaschen und schliesslich mittels Säulenchromatographie an Kieselgel (Laufmittel: Äthylacetat/Hexan - 1:3) gereinigt. Man erhält die Titelverbindung der Formel

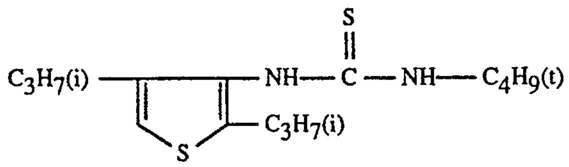

in Form eines farblosen Kristallpulvers vom Smp. 168°-169°C (Verbindung Nr. 1.01).

In analoger Weise werden die folgenden erfindungsgemässen Thioharnstoffe der Formel Ia hergestellt:

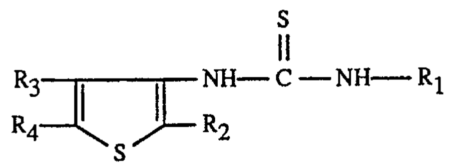

(Ia)

| Verbindung Nr. | $R_1$ | $R_2$ | $R_3$ | $R_4$ | Smp. (°C) |
|---|---|---|---|---|---|
| 1.02 | $C_3H_7$-i | H | H | $C_6H_5$ | 151-154 |
| 1.03 | $C_4H_9$-t | H | H | $C_6H_5$ | 130-133 |
| 1.04 | $C(CH_3)_2C_2H_5$ | H | H | $C_6H_5$ | 124-125 |
| 1.05 | $C_5H_9$-cyclo | H | H | $C_6H_5$ | 110-113 |
| 1.06 | $C_4H_9$-t | H | H | $CH_3$ | 102-105 |
| 1.07 | $C_4H_9$-t | H | $CH_3$ | $CH_3$ | 143-146 |
| 1.08 | $C_3H_7$-i | H | $CH_3$ | $CH_3$ | 120-122 |
| 1.09 | $C_4H_9$-t | $CH_3$ | $CH_3$ | $CH_3$ | 169-170 |
| 1.10 | $C_3H_7$-i | $CH_3$ | $CH_3$ | $CH_3$ | 125-126 |
| 1.11 | $C_4H_9$-t | $CH_3$ | H | $C_6H_5$ | 153-154 |
| 1.12 | $C_4H_9$-t | $CH_3$ | $C_3H_7$-i | $C_4H_9$-i | 87- 90 |
| 1.13 | $C_3H_7$-i | $CH_3$ | $C_3H_7$-i | $C_4H_9$-i | 93- 95 |
| 1.14 | $C_4H_9$-t | $C_3H_7$-i | H | $C_6H_5$ | 157-158 |
| 1.15 | $C_3H_7$-i | $C_3H_7$-i | H | $C_6H_5$ | 155-156 |
| 1.16 | $C_4H_9$-t | $C_3H_7$-i | H | $CH_3$ | 122-123 |
| 1.17 | $C_3H_7$-i | $C_3H_7$-i | H | $CH_3$ | 125-127 |
| 1.18 | $C_4H_9$-t | $C_3H_7$-i | $CH_3$ | $CH_3$ | 95- 96 |
| 1.19 | $C_3H_7$-i | $C_3H_7$-i | $CH_3$ | $CH_3$ | 101-103 |
| 1.20 | $C_3H_7$-i | $C_3H_7$-i | $C_3H_7$-i | H | 171-172 |
| 1.21 | $C_4H_9$-t | $C_2H_5$ | $C_3H_7$-i | H | 92- 94 |

Wie vorstehend angegeben sind auch die folgenden Verbindungen der Formel Ia herstellbar:

| $R_1$ | $R_2$ | $R_3$ | $R_4$ |
|---|---|---|---|
| $C(CH_3)_2$-$C_2H_5$ | $C_3H_7$-i | $C_3H_7$-i | H |
| $C_5H_9$-cyclo | $C_3H_7$-i | $C_3H_7$-i | H |
| $C_3H_7$-i | $C_2H_5$ | $C_3H_7$-i | H |

Beispiel 3: Herstellung von 1-tert.Butyl-3-(2,4-diisopropyl-3-thienyl)-S-methylisothioharnstoff

1,50 g des nach Beispiel 2 erhaltenen 1-tert.Butyl-3-(2,4-diisopropyl-3-thienyl)thioharnstoffes in 30 ml Äthanol werden bei Raumtemperatur mit 1,65 g Methyljodid versetzt und während 5 Stunden auf +75 °C erwärmt. Anschliessend wird die Mischung eingedampft, der Rückstand in Dichlormethan aufgenommen und zweimal mit verdünnter Natriumbicarbonatlösung gewaschen. Die abgetrennte organische Phase wird über Magnesiumsulfat getrocknet und das Lösungsmittel abgedampft. Das Rohprodukt wird mittels Säulenchromatographie an Kieselgel (Laufmittel: Äthylacetat/Hexan - 1:10) gereinigt. Man erhält so die Titelverbindung der Formel

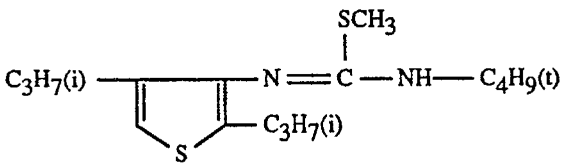

in Form eines farblosen, dickflüssigen Öls, $n_D^{22}$ = 1,5404 (Verbindung Nr. 2.01).

In analoger Weise werden auch die folgenden erfindungsgemässen Isothioharnstoffe der Formel Ib hergestellt:

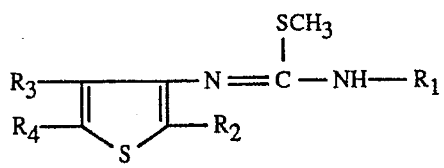

(Ib)

| Verbindung Nr. | $R_1$ | $R_2$ | $R_3$ | $R_4$ | Phys. Daten |
|---|---|---|---|---|---|
| 2.02 | $C_3H_7$-i | H | H | $C_6H_5$ | $n_D^{22} = 1,6445$ |
| 2.03 | $C_4H_9$-t | H | H | $C_6H_5$ | Smp. 51-54°C |
| 2.04 | $C(CH_3)_2C_2H_5$ | H | H | $C_6H_5$ | $n_D^{23} = 1,6205$ |
| 2.05 | $C_5H_9$-cyclo | H | H | $C_6H_5$ | $n_D^{23} = 1,6480$ |
| 2.06 | $C_4H_9$-t | H | H | $CH_3$ | |
| 2.07 | $C_4H_9$-t | H | $CH_3$ | $CH_3$ | $n_D^{24} = 1,5665$ |
| 2.08 | $C_3H_7$-i | H | $CH_3$ | $CH_3$ | Smp. 50-53°C |
| 2.09 | $C_4H_9$-t | $CH_3$ | $CH_3$ | $CH_3$ | $n_D^{23} = 1,5520$ |
| 2.10 | $C_3H_7$-i | $CH_3$ | $CH_3$ | $CH_3$ | $n_D^{23} = 1,5625$ |
| 2.11 | $C_4H_9$-t | $CH_3$ | H | $C_6H_5$ | |
| 2.12 | $C_4H_9$-t | $CH_3$ | $C_3H_7$-i | $C_4H_9$-i | $n_D^{25} = 1,5378$ |
| 2.13 | $C_3H_7$-i | $CH_3$ | $C_3H_7$-i | $C_4H_9$-i | $n_D^{25} = 1,5380$ |
| 2.14 | $C_4H_9$-t | $C_3H_7$-i | H | $C_6H_5$ | $n_D^{23} = 1,6045$ |
| 2.15 | $C_3H_7$-i | $C_3H_7$-i | H | $C_6H_5$ | Smp. 83-86°C |
| 2.16 | $C_4H_9$-t | $C_3H_7$-i | H | $CH_3$ | $n_D^{23} = 1,5495$ |
| 2.17 | $C_3H_7$-i | $C_3H_7$-i | H | $CH_3$ | $n_D^{23} = 1,5555$ |
| 2.18 | $C_4H_9$-t | $C_3H_7$-i | $CH_3$ | $CH_3$ | Smp. 63-66°C |
| 2.19 | $C_3H_7$-i | $C_3H_7$-i | $CH_3$ | $CH_3$ | $n_D^{25} = 1,5470$ |
| 2.20 | $C_4H_9$-t | $C_2H_5$ | $C_3H_7$-i | H | $n_D^{20} = 1,5312$ |
| 2.21 | $C_3H_7$-i | $C_3H_7$-i | $C_3H_7$-i | H | $n_D^{23} = 1,5421$ |

Wie vorstehend angegeben sind auch die folgenden Verbindungen der Formel Ib herstellbar:

| $R_1$ | $R_2$ | $R_3$ | $R_4$ |
|---|---|---|---|
| $C(CH_3)_2$-$C_2H_5$ | $C_3H_7$-i | $C_3H_7$-i | H |
| $C_5H_9$-cyclo | $C_3H_7$-i | $C_3H_7$-i | H |
| $C_3H_7$-i | $C_2H_5$ | $C_3H_7$-i | H |

Beispiel 4: Herstellung des Oxalsäuresalzes des 1-tert.Butyl-3-(2-isopropyl-5-methyl-3-thienyl)-S-methylisothioharnstoffes

Es werden 0,85 g des nach Beispiel 3 erhaltenen 1-tert.Butyl-3-(2-isopropyl-5-methyl-3-thienyl)-S-methylisothioharnstoffes in 1 ml Äther (Diäthyläther) gelöst und mit 3,5 ml einer gesättigten Oxalsäure-Lösung in Äther unter Rühren versetzt. Nach Verdünnen mit 10 ml Äther wird das Gemisch während 15 Minuten bei Raumtemperatur weiter gerührt. Der entstandene Festkörper wird abgenutscht und dreimal mit 10 ml Äther gründlich nachgewaschen. Die Titelverbindung liegt in Form eines farblosen Kristallpulvers, Smp. 138°-140°C (Verbindung Nr. 3.01), vor.

In analoger Weise werden die folgenden erfindungsgemässen Isothioharnstoff-Salze der Formel Ic hergestellt:

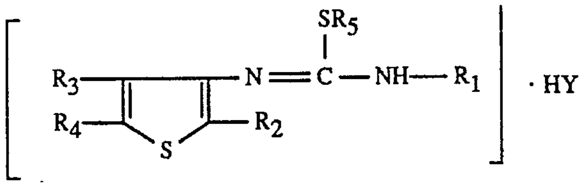

(Ic)

| Verb. Nr. | $R_1$ | $R_2$ | $R_3$ | $R_4$ | $R_5$ | Y | Smp.(°C) |
|---|---|---|---|---|---|---|---|
| 3.02 | $C_3H_7$-i | $C_3H_7$-i | H | $C_6H_5$ | $CH_3$ | $H(COO)_2$ | 200-202 |
| 3.03 | $C_3H_7$-i | $CH_3$ | $CH_3$ | $CH_3$ | $CH_3$ | $H(COO)_2$ | 175-178 |
| 3.04 | $C_4H_9$-t | $CH_3$ | $CH_3$ | $CH_3$ | $CH_3$ | $H(COO)_2$ | 110-112 |

Herstellbar sind in analoger Weise auch folgende Salze der Formel Ic:

| $R_1$ | $R_2$ | $R_3$ | $R_4$ | $R_5$ | Y |
|---|---|---|---|---|---|
| $C_4H_9$-t | $C_3H_7$-i | $C_3H_7$-i | H | $CH_3$ | $H(COO)_2$ |
| $C_3H_7$-i | $C_3H_7$-i | $CH_3$ | $CH_3$ | $CH_3$ | $H(COO)_2$ |
| $C_4H_9$-t | $C_2H_5$ | $C_3H_7$-i | H | $CH_3$ | $H(COO)_2$ |

Beispiel 5: Herstellung von 1-tert.Butyl-3-(2,4-diisopropyl-3-thienyl)-carbodiimid

Es werden 2,50 g des nach Beispiel 2 erhaltenen 1-tert.Butyl-3-(2,4-diisopropyl-3-thienyl)thioharnstoffes

und 2,60 g 2-Chlor-1-methylpyridiniumjodid in 25 ml Acetonitril vorgelegt und bei Raumtemperatur mit einer Lösung von 1,90 g Triäthylamin in 12 ml Acetonitril versetzt. Anschliessend wird das Reaktionsgemisch während 2 Stunden bei ca. +45° C verrührt. Nach dem Abdampfen des Lösungsmittels wird der Rückstand in Hexan/Wasser aufgenommen. Die abgetrennte organische Phase wird über Magnesiumsulfat getrocknet und das Lösungsmittel am Vakuum entfernt. Das Rohprodukt wird mittels Säulenchromatographie an Kieselgel (Laufmittel: Äthylacetat/Hexan - 1:20) gereinigt. Man erhält die Titelverbindung der Formel

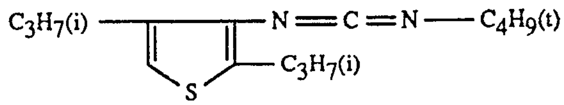

als farbloses Öl $n_D^{23}$ = 1,5249 (Verbindung Nr. 4.01).

In analoger Weise werden die folgenden erfindungsgemässen Carbodiimide der Formel Id hergestellt:

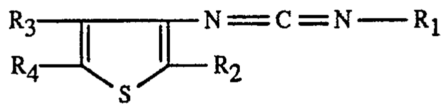

(Id)

| Verbindung Nr. | $R_1$ | $R_2$ | $R_3$ | $R_4$ | Phys. Daten |
|---|---|---|---|---|---|
| 4.02 | $C_3H_7$-i | H | H | $C_6H_5$ | $n_D^{23} = 1,6185$ |
| 4.03 | $C_4H_9$-t | H | H | $C_6H_5$ | $n_D^{23} = 1,6040$ |
| 4.04 | $C(CH_3)_2C_2H_5$ | H | H | $C_6H_5$ | $n_D^{24} = 1,5955$ |
| 4.05 | $C_5H_9$-cyclo | H | H | $C_6H_5$ | wachsartige Masse |
| 4.06 | $C_4H_9$-t | H | $CH_3$ | $CH_3$ | $n_D^{24} = 1,5360$ |
| 4.07 | $C_4H_9$-t | $CH_3$ | $CH_3$ | $CH_3$ | $n_D^{24} = 1,5335$ |
| 4.08 | $C_3H_7$-i | $CH_3$ | $CH_3$ | $CH_3$ | $n_D^{24} = 1,5375$ |
| 4.09 | $C_4H_9$-t | $CH_3$ | $C_3H_7$-i | $C_4H_9$-i | $n_D^{25} = 1,5215$ |
| 4.10 | $C_3H_7$-i | $CH_3$ | $C_3H_7$-i | $C_4H_9$-i | $n_D^{25} = 1,5323$ |
| 4.11 | $C_4H_9$-t | $C_3H_7$-i | H | $C_6H_5$ | $n_D^{23} = 1,5875$ |
| 4.12 | $C_3H_7$-i | $C_3H_7$-i | H | $C_6H_5$ | $n_D^{23} = 1,5985$ |
| 4.13 | $C_4H_9$-t | $C_3H_7$-i | H | $CH_3$ | $n_D^{23} = 1,5235$ |
| 4.14 | $C_3H_7$-i | $C_3H_7$-i | H | $CH_3$ | $n_D^{23} = 1,5285$ |
| 4.15 | $C_4H_9$-t | $C_3H_7$-i | $CH_3$ | $CH_3$ | $n_D^{24} = 1,5265$ |
| 4.16 | $C_3H_7$-i | $C_3H_7$-i | $CH_3$ | $CH_3$ | $n_D^{24} = 1,5335$ |
| 4.17 | $C_4H_9$-t | $C_2H_5$ | $C_3H_7$-i | H | $n_D^{20} = 1,5490$ |
| 4.18 | $C_3H_7$-i | $C_3H_7$-i | $C_3H_7$-i | H | $n_D^{23} = 1,5309$ |

Wie vorstehend angegeben sind auch die folgenden Verbindungen der Formel Id herstellbar:

| $R_1$ | $R_2$ | $R_3$ | $R_4$ |
|---|---|---|---|
| $C(CH_3)_2$-$C_2H_5$ | i-$C_3H_7$ | $C_3H_7$-i | H |
| $C_5H_9$-cyclo | i-$C_3H_7$ | $C_3H_7$-i | H |
| $C_3H_7$-i | $C_2H_5$ | $C_3H_7$-i | H |
| $C_4H_9$-t | H | H | $CH_3$ |
| $C_3H_7$-i | H | $CH_3$ | $CH_3$ |
| $C_4H_9$-t | $CH_3$ | H | $C_6H_5$ |

Beispiel 6: Formulierungen von Wirkstoffen der Formel I gemäss den Herstellungsbeispielen 2 bis 5 (% = Gewichtsprozent)

| Beispiel 6.1: Emulsions-Konzentrate | a) | b) | c) |
|---|---|---|---|
| Wirkstoff (Verb.Nr. 1.01-1.21; 2.01-2.21; 3.01-3.04; 4.01-4.18) | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel 6.2: Lösungen | a) | b) | c) | d) |
|---|---|---|---|---|
| Wirkstoff (Verb.Nr. 1.01-1.21; 2.01-2.21; 3.01-3.04; 4.01-4.18) | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

| Beispiel 6.3: Granulate | a) | b) |
|---|---|---|
| Wirkstoff (Verb.Nr. 1.01-1.21; 2.01-2.21; 3.01-3.04; 4.01-4.18) | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Beispiel 6.4: Stäubemittel | a) | b) |
|---|---|---|
| Wirkstoff (Ver.Nr. 1.01-1.21; 2.01-2.21; 3.01-3.04; 4.01-4.18) | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel 6.5: Spritzpulver | a) | b) | c) |
|---|---|---|---|
| Wirkstoff (Ver.Nr. 1.01-1.21; 2.01-2.21; 3.01-3.04; 4.01-4.18) | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel 6.6: Emulsions-Konzentrat | |
|---|---|
| Wirkstoff (Verb.Nr. 1.01-1.21; 2.01-2.21; 3.01-3.04; 4.01-4.18) | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel 6.7: Stäubemittel | a) | b) |
|---|---|---|
| Wirkstoff (Verb.Nr. 1.01-1.21; 2.01-2.21; 3.01-3.04; 4.01-4.18) | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel 6.8: Extruder-Granulat | |
|---|---|
| Wirkstoff (Verb.Nr. 1.01-1.21; 2.01-2.21; 3.01-3.04; 4.01-4.18) | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| Beispiel 6.9: Umhüllungs-Granulat | |
|---|---|
| Wirkstoff (Verb.Nr. 1.01-1.21; 2.01-2.21; 3.01-3.04; 4.01-4.18) | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| Beispiel 6.10: Suspensions-Konzentrat | |
|---|---|
| Wirkstoff (Verb.Nr. 1.01-1.21; 2.01-2.21; 3.01-3.04; 4.01-4.18) | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 7: Wirkung gegen Boophilus microplus

Gut ernährte Zecken Weibchen werden auf eine PVC Platte aufgeklebt und mit einem Wattebausch überdeckt. Zur Behandlung werden die Testtiere mit 10 ml einer wässrigen Testlösung, die 400 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen der Formel I gemäss den Beispielen 2 bis 5 zeigen in diesem Test gute Wirkung gegen Boophilus microplus. Insbesondere die Verbindungen Nr. 4.01, 4.11 und 4.13 zeigen eine Wirkung über 80 %.

Beispiel 8: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reis pflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Formel I gemäss den Beispielen 2 bis 5 zeigen eine gute Wirkung gegen Nilaparvata lugens in diesem Test. Insbesondere die Verbindungen Nr. 1.01, 1.14, 1.16, 1.18, 2.01, 2.14, 2.18, 4.01, 4.11 und 4.15 zeigen eine Wirkung über 80 %.

Beispiel 9: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Formel I gemäss den Beispielen 2 bis 5 zeigen eine gute Wirkung gegen Nephotettix cincticeps in diesem Test. Insbesondere die Verbindungen Nr. 1.01, 1.18, 2.01, 2.18 und 4.15 zeigen eine Wirkung über 80 %.

Beispiel 10: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu unbehandelten Kontrollansätzen.

Verbindungen der Formel I gemäss den Beispielen 2 bis 5 zeigen in diesem Test gute Wirkung gegen Bemisia tabaci. Insbesondere die Verbindungen Nr. 1.01 und 4.01 zeigen eine Wirkung über 80 %.

Beispiel 11: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Die Verbindungen der Formel I gemäss den Beispielen 2 bis 5 zeigen eine gute Wirkung gegen Diabrotica balteata in diesem Test. Die Verbindungen Nr. 1.01, 1.09 und 4.01 zeigen eine Wirkung über 80 %. Die Verbindung 2.01 zeigt 100%-ige Wirkung.

Beispiel 12: Wirkung gegen Tetranychus urticae

Junge Bohnenpflanzen werden mit einer Mischpopulation von Tetranychus urticae besiedelt und 1 Tag später mit einer wässrigen Emulsions- Spritzbrühe, die 400 ppm des Wirkstoffes enthält, besprüht. Die Pflanzen werden anschliessend für 6 Tage bei 25 oC inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Die Verbindungen der Formel I gemäss den Beispielen 2 bis 5 zeigen eine gute Wirkung gegen Tetranychus urticae in diesem Test. Insbesondere die Verbindungen Nr. 1.01, 1.14, 1.15, 1.18, 4.01 und 4.04 zeigen eine Wirkung über 80 %.

Beispiel 13: Wirkung gegen Spodoptera littoralis Raupen

Junge Sojapflanzen werden mit einer wässrigen Emulsions- Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Formel I gemäss den Beispielen 2 bis 5 zeigen eine gute Wirkung gegen Spodoptera littoralis in diesem Test. Insbesondere die Verbindungen Nr. 1.16, 1.18, 1.19, 2.04, 2.14 und 4.11 zeigen eine Wirkung über 80 %.

Beispiel 14: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20 °C inkubiert. Nach 3 und 6 Tagen erfolgen die Auswertungen. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Formel I gemäss den Beispielen 2 bis 5 zeigen eine gute Wirkung gegen Aphis craccivora in diesem Test. Insbesondere die Verbindungen Nr. 1.01, 4.01, 4.11 und 4.15 zeigen eine Wirkung über 80 %.

Beispiel 15: Wirkung gegen Tetranychus cinnabarinus (OP-resistant)

Die Primärblätter von Phaseolus vulgaris-Pflanzen werden 24 Stunden vor dem Versuch auf akarizide Wirkung mit einem infestierten Blattstück aus einer Massenzucht von Tetranychus cinnabarinus belegt (die Resistenz bezieht sich auf die Verträglichkeit gegenüber Diazinon).

Die so behandelten infestierten Pflanzen werden mit einer wässrigen Zubereitung enthaltend 400 ppm der jeweils zu prüfenden Verbindung bis zur Tropfnässe besprüht. Nach 48 Stunden und wiederum nach 7 Tagen werden Imagines und Larven (alle beweglichen Stadien) unter dem Binokular auf lebende und tote Individuen ausgewertet. Während des Versuchsverlaufs stehen die Pflanzen in Gewächshauskabinen bei 25 °C.

Verbindungen der Formel I gemäss den Beispielen 2 bis 5 zeigen in diesem Versuch gute Wirkung gegen Tetranychus cinnabarinus. So wird mit den Verbindungen Nr. 1.14, 1.18 und 4.01 eine Wirkung von 80-100 % erzielt.

Beispiel 16: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispielen 2 bis 5 zeigen gute Wirkung im obigen Test.

Beispiel 17: Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt.

Verbindungen der Formel I gemäss den Beispielen 2 bis 5 zeigen eine gute Wirkung gegen Dermanyssus gallinae.

Beispiel 18: Wirkung gegen Blattella germanica

In eine Petri-Schale von 10 cm Durchmesser wird so viel einer 0,1%igen acetonischen Lösung des Wirkstoffes gegeben, dass die Menge einer Aufwandmenge von 1 $g/m^2$ ent spricht. Wenn das Lösungsmittel verdunstet ist, werden 10 Blattella germanica Nymphen (letztes Nymphenstadium) in die so vorbereitete Schale gegeben und 2 Stunden lang der Wirkung der Testsubstanz ausgesetzt. Dann werden die Nymphen mit $CO_2$ narkotisiert, in eine frische Petri-Schale gebracht und im Dunkeln bei 25 °C und ca. 70 % Luftfeuchtigkeit gehalten. Nach 48 Stunden wird die insektizide Wirkung durch Ermittlung der Abtötungsrate bestimmt.

Verbindungen der Formel I gemäss den Beispielen 2 bis 5 zeigen eine gute Wirkung im obigen Test.

## Ansprüche

1. Verbindung der Formel I

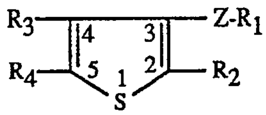

(I),

worin

$R_1$ $C_1$-$C_{12}$-Alkyl, durch einen $C_3$-$C_6$-Cycloalkylrest substituiertes $C_1$-$C_4$-Alkyl, $C_3$-$C_8$-Cycloalkyl oder durch einen $C_1$-$C_4$-Alkylrest substituiertes $C_3$-$C_6$-Cycloalkyl;

$R_2$, $R_3$ und $R_4$ unabhängig voneinander Wasserstoff, $C_1$-$C_6$-Alkyl, Phenyl oder durch 1 bis 3 Reste aus der Gruppe Halogen, $C_1$-$C_6$-Alkyl, $C_1$-$C_4$-Alkoxy und $C_1$-$C_6$-Halogenalkyl mit 1 bis 9 Halogenatomen substituiertes Phenyl;

Z eine Gruppe -NH-CS-NH-, -N=C($SR_5$)-NH- oder -N=C=N-; und $R_5$ $C_1$-$C_6$-Alkyl oder $C_3$-$C_5$-Alkenyl;

bedeuten; mit der Massgabe, dass mindestens einer der Reste $R_2$, $R_3$ oder $R_4$ eine von Wasserstoff abweichende Bedeutung hat; sowie die Salze von Verbindungen der Formel I, worin Z die Gruppe -N=C-($SR_5$)-NH- bedeutet.

2. Verbindung gemäss Anspruch 1, dadurch gekennzeichnet, dass mindestens zwei der Reste $R_2$, $R_3$ oder $R_4$ eine von Wasserstoff abweichende Bedeutung haben.

3. Verbindung gemäss Anspruch 1 oder 2, dadurch gekennzeichnet, dass $R_4$ Wasserstoff bedeutet.

4. Verbindung gemäss einem der Ansprüche 1 bis 3, dadurch gekennzeichnet, dass $R_1$ $C_1$-$C_4$-Alkyl oder $C_3$-$C_5$-Cycloalkyl bedeutet.

5. Verbindung gemäss Anspruch 4, dadurch gekennzeichnet, dass $R_1$ $C_4H_9$(t) oder $C_3H_7$(i) bedeutet.

6. Verbindung gemäss einem der Ansprüche 1 bis 5, dadurch gekennzeichnet, dass $R_2$ und $R_3$ unabhängig voneinander $C_2$-$C_5$-Alkyl bedeuten.

7. Verbindung gemäss einem der Ansprüche 1 bis 6, dadurch gekennzeichnet, dass $R_2$ und/oder $R_3$ $C_3H_7$(i) bedeuten.

8. Verbindung gemäss einem der Ansprüche 1 bis 7, dadurch gekennzeichnet, dass $R_5$ Methyl oder Aethyl bedeutet.

9. Verbindung gemäss Anspruch 7 der Formel

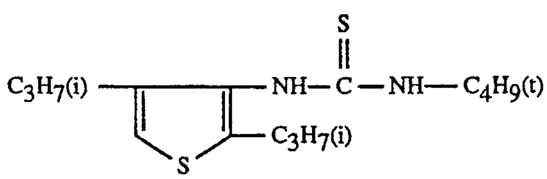

.

10. Verbindung gemäss Anspruch 7 der Formel

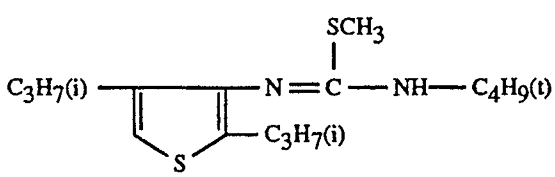

.

11. Verbindung gemäss Anspruch 7 der Formel

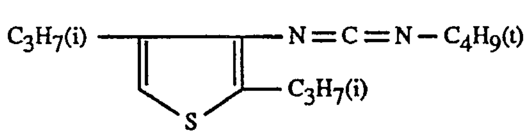

.

12. Verbindung gemäss Anspruch 7 der Formel

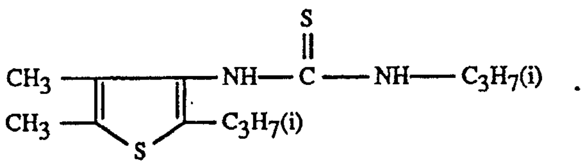

13. Verbindung gemäss Anspruch 7 der Formel

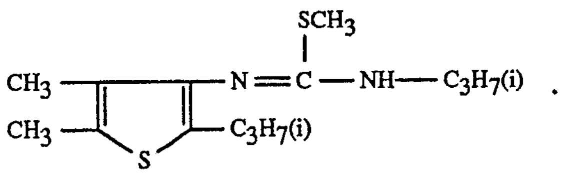

14. Verbindung gemäss Anspruch 7 der Formel

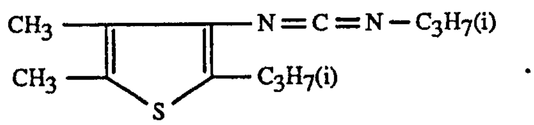

15. Verfahren zur Herstellung einer Verbindung der Formel I

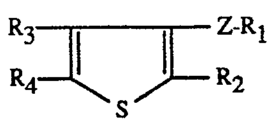

(I),

worin
$R_1$ $C_1$-$C_{12}$-Alkyl, durch einen $C_3$-$C_6$-Cycloalkylrest substituiertes $C_1$-$C_4$-Alkyl, $C_3$-$C_8$-Cycloalkyl oder durch einen $C_1$-$C_4$-Alkylrest substituiertes $C_3$-$C_6$-Cycloalkyl;
$R_2$, $R_3$ und $R_4$ unabhängig voneinander Wasserstoff, $C_1$-$C_6$-Alkyl, Phenyl oder durch 1 bis 3 Reste aus der Gruppe Halogen, $C_1$-$C_6$-Alkyl, $C_1$-$C_4$-Alkoxy und $C_1$-$C_6$-Halogenalkyl mit 1 bis 8 Halogenatomen substituiertes Phenyl;
Z eine Gruppe -NH-CS-NH-, -N=C($SR_5$)-NH- oder -N=C=N-; und
$R_5$ $C_1$-$C_6$-Alkyl oder $C_3$-$C_5$-Alkenyl;
bedeuten; mit der Massgabe, dass mindestens einer der Reste $R_2$, $R_3$ oder $R_4$ eine von Wasserstoff abweichende Bedeutung hat, sowie der Salze von Verbindungen der Formel I, worin Z die Gruppe -N=C-($SR_5$)-NH- bedeutet; dadurch gekennzeichnet, dass man
a) zur Herstellung einer Verbindung der Formel I, worin Z für -NH-CS-NH- steht, eine Verbindung der Formel II

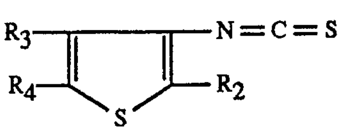

(II)

mit einer Verbindung der Formel III
$H_2N$-$R_1$ (III)
umsetzt; oder
b) gegebenenfalls zur Herstellung einer Verbindung der Formel I, worin Z für -N=C($SR_5$)-NH- steht, die erhaltene Verbindung der Formel I, worin Z für -NH-CS-NH-steht, mit einer Verbindung der Formel IV
X-$R_1$ (IV)
umsetzt und gegebenenfalls eine erhaltene Verbindung der Formel I, worin Z für -N=C($SR_5$)-NH- steht, in

ein Salz überführt; oder
c) gegebenenfalls zur Herstellung einer Verbindung der Formel I, worin Z für -N=C=N-steht, aus einer erhaltenen Verbindung der Formel I, worin Z für -NH-CS-NH- steht, Schwefelwasserstoff abspaltet;
wobei in den Formeln II bis IV $R_1$ bis $R_5$ die oben angegebenen Bedeutungen haben und X für eine Abgangsgruppe steht.
16. Verbindung der Formel II

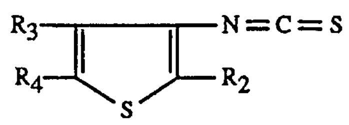
(II),

worin $R_2$ bis $R_4$ die in Anspruch 1 angegebenen Bedeutungen haben.
17. Schädlingsbekämpfungsmittel, welches als aktive Komponente eine Verbindung gemäss einem der Ansprüche 1 bis 14 zusammen mit geeigneten Trägern und/oder anderen Zuschlagsstoffen enthält.
18. Verwendung einer Verbindung gemäss einem der Ansprüche 1 bis 14 zur Bekämpfung von Insekten und Vertretern der Ordnung Akarina an Tieren und Pflanzen.
19. Verwendung gemäss Anspruch 18 zur Bekämpfung von pflanzenschädigenden Insekten.
20. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 14 oder mit einem Mittel enthaltend neben Zusatz- und Trägerstoffen eine pestizid wirksame Menge dieser Verbindung, in Kontakt bringt oder behandelt.

Patentansprüche für folgenden Vertragsstaat: ES

1. Verfahren zur Herstellung einer Verbindung der Formel I

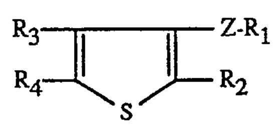
(I),

worin
$R_1$ $C_1$-$C_{12}$-Alkyl, durch einen $C_3$-$C_6$-Cycloalkylrest substituiertes $C_1$-$C_4$-Alkyl, $C_3$-$C_8$-Cycloalkyl oder durch einen $C_1$-$C_4$-Alkylrest substituiertes $C_3$-$C_6$-Cycloalkyl;
$R_2$, $R_3$ und $R_4$ unabhängig voneinander Wasserstoff, $C_1$-$C_6$-Alkyl, Phenyl oder durch 1 bis 3 Reste aus der Gruppe Halogen, $C_1$-$C_6$-Alkyl, $C_1$-$C_4$-Alkoxy und $C_1$-$C_6$-Halogenalkyl mit 1 bis 8 Halogenatomen substituiertes Phenyl;
Z eine Gruppe -NH-CS-NH-, -N=C($SR_5$)-NH- oder -N=C=N-; und
$R_5$ $C_1$-$C_6$-Alkyl oder $C_3$-$C_5$-Alkenyl;
bedeuten; mit der Massgabe, dass mindestens einer der Reste $R_2$, $R_3$ oder $R_4$ eine von Wasserstoff abweichende Bedeutung hat, sowie der Salze von Verbindungen der Formel I, worin Z die Gruppe -N=C-($SR_5$)-NH- bedeutet; dadurch gekennzeichnet, dass man
a) zur Herstellung einer Verbindung der Formel I, worin Z für -NH-CS-NH- steht, eine Verbindung der Formel II

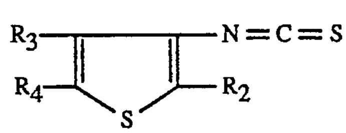
(II)

mit einer Verbindung der Formel III
$H_2N$-$R_1$ (III)
umsetzt; oder
b) gegebenenfalls zur Herstellung einer Verbindung der Formel I, worin Z für -N=C($SR_5$)-NH- steht, die

erhaltene Verbindung der Formel I, worin Z für -NH-CS-NH-steht, mit einer Verbindung der Formel IV

$X\text{-}R_1$ (IV)

umsetzt und gegebenenfalls eine erhaltene Verbindung der Formel I, worin Z für $-N=C(SR_5)\text{-}NH\text{-}$ steht, in ein Salz überführt; oder

c) gegebenenfalls zur Herstellung einer Verbindung der Formel I, worin Z für $-N=C=N$-steht, aus einer erhaltenen Verbindung der Formel I, worin Z für -NH-CS-NH- steht, Schwefelwasserstoff abspaltet;

wobei in den Formeln II bis IV $R_1$ bis $R_5$ die oben angegebenen Bedeutungen haben und X für eine Abgangsgruppe steht.

2. Verfahren gemäss Anspruch 1 zur Herstellung einer Verbindung der Formel I, worin mindestens zwei der Reste $R_2$, $R_3$ oder $R_4$ eine von Wasserstoff abweichende Bedeutung haben.

3. Verfahren gemäss Anspruch 1 oder 2 zur Herstellung einer Verbindung der Formel I, worin $R_4$ Wasserstoff bedeutet.

4. Verfahren gemäss einem der Ansprüche 1 bis 3 zur Herstellung einer Verbindung der Formel I, worin $R_1$ $C_1$-$C_4$-Alkyl oder $C_3$-$C_5$-Cycloalkyl bedeutet.

5. Verfahren gemäss Anspruch 4 zur Herstellung einer Verbindung der Formel I, worin $R_1$ $C_4H_9$(t) oder $C_3H_7$(i) bedeutet.

6. Verfahren gemäss einem der Ansprüche 1 bis 5 zur Herstellung einer Verbindung der Formel I, worin $R_2$ und $R_3$ unabhängig voneinander $C_2$-$C_5$-Alkyl bedeuten.

7. Verfahren gemäss einem der Ansprüche 1 bis 6 zur Herstellung einer Verbindung der Formel I, worin $R_2$ und/oder $R_3$ $C_3H_7$(i) bedeuten.

8. Verfahren gemäss einem der Ansprüche 1 bis 7 zur Herstellung einer Verbindung der Formel I, worin $R_5$ Methyl oder Aethyl bedeutet.

9. Verfahren zur Herstellung einer Verbindung gemäss Anspruch 7 der Formel

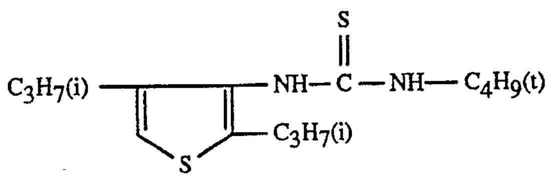 .

10. Verfahren zur Herstellung einer Verbindung gemäss Anspruch 7 der Formel

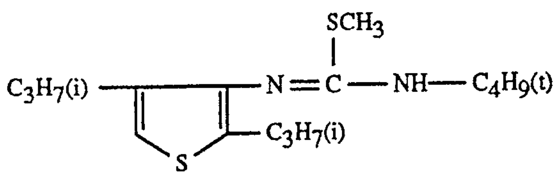 .

11. Verfahren zur Herstellung einer Verbindung gemäss Anspruch 7 der Formel

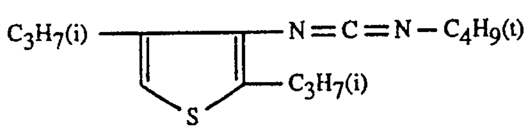 .

12. Verfahren zur Herstellung einer Verbindung gemäss Anspruch 7 der Formel

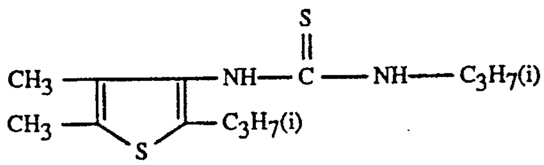 .

13. Verfahren zur Herstellung einer Verbindung gemäss Anspruch 7 der Formel

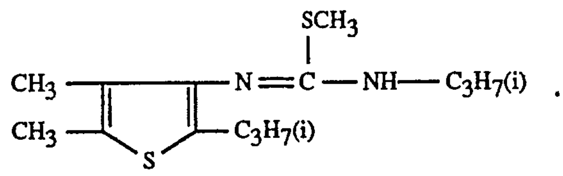

14. Verfahren zur Herstellung einer Verbindung gemäss Anspruch 7 der Formel

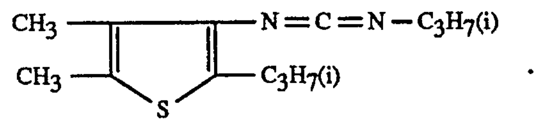

15. Schädlingsbekämpfungsmittel, welches als aktive Komponente eine Verbindung gemäss einem der Ansprüche 1 bis 14 zusammen mit geeigneten Trägern und/oder anderen Zuschlagsstoffen enthält.

16. Verwendung einer Verbindung gemäss einem der Ansprüche 1 bis 14 zur Bekämpfung von Insekten und Vertretern der Ordnung Akarina an Tieren und Pflanzen.

17. Verwendung gemäss Anspruch 16 zur Bekämpfung von pflanzenschädigenden Insekten.

18. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 14 oder mit einem Mittel enthaltend neben Zusatz- und Trägerstoffen eine pestizid wirksame Menge dieser Verbindung, in Kontakt bringt oder behandelt.